Europäisches Patentamt

European Patent Office

Office européen des brevets

(19)

(11) Veröffentlichungsnummer: **0 202 511**
**B1**

(12) **EUROPÄISCHE PATENTSCHRIFT**

(45) Veröffentlichungstag der Patentschrift:
07.06.89

(51) Int. Cl.⁴: **C 07 D277/80**

(21) Anmeldenummer: **86105858.4**

(22) Anmeldetag: **28.04.86**

(54) Verfahren zur Herstellung von Benzothiazolsulfenamiden.

(30) Priorität: 11.05.85 DE 3517084

(43) Veröffentlichungstag der Anmeldung:
26.11.86 Patentblatt 86/48

(45) Bekanntmachung des Hinweises auf die Patenterteilung: 07.06.89 Patentblatt 89/23

(84) Benannte Vertragsstaaten:
BE DE FR GB IT NL

(56) Entgegenhaltungen:
EP–A– 0 010 477
EP–A– 0 173 871
GB–A– 737 252
CHEMICAL ABSTRACTS, Band 92, Nr. 9, 3. März 1980, Seite 667, Nr. 76380u, Columbus, Ohio, US; V.A. IGNATOV et al.: "Study of the reaction by bis(2-benzothiazolyl)disulfide with amines"

(73) Patentinhaber: BAYER AG
D-5090 Leverkusen 1 Bayerwerk (DE)

(72) Erfinder: Wüst, Alfredo, Dr.
Siebengebirgsweg 14
D-5064 Rösrath (DE)
Erfinder: van Osselaer, Tony, Dr.
Tuinlaan 62
B-9180 Belsele (BE)

## Beschreibung

Die Erfindung betrifft ein Verfahren zur Herstellung von Benzothiazolsulfenamiden aus Dibenzothiazolyldisulfiden und wasserlöslichen Aminen.

Benzothiazolsulfenamide werden üblicherweise durch oxidative Kupplung von 2-Mercaptobenzothiazolen, deren Alkalisalzen oder Disulfiden und primären oder sekundären Aminen hergestellt (Deutsche Offenlegungsschriften 2 356 686 und 2 744 423). Dabei werden entweder nur mäßige Ausbeuten in Bezug auf die Mercaptobenzothiazol-Basis erzielt und die Produkte sind durch Nebenprodukte verunreinigt, die die Lagerstabilität des Sulfenamids beeinträchtigen, oder es müssen aufwendige und unökonomische Verfahren mit komplizierten Reinigungsschritten angewendet werden.

Auf das Amin bezogen werden nach den bekannten Verfahren Ausbeuten von höchstens 90 % erreicht. Dies ist ein wesentlicher Nachteil, da für die Sulfenamide, die bekanntlich als Vulkanisationsbeschleuniger eingesetzt werden, Amine Verwendung finden, deren Herstellung mindestens ebenso kostspielig ist wie die Herstellung des Mercaptobenzothiazolteils.

Es ist weiterhin aus Izv. vyssh. Uchebn. Zaved., Khimiya i khim. tekhnol., 22 (1979), 9, 1067-1070 bekannt, Dibenzothiazolyldisulfid (MBTS) mit Aminen in An- und Abwesenheit von Alkali und in Abwesenheit eines Oxidationsmittels umzusetzen.

Ohne Alkali wurde mit Morpholin als Amin eine Ausbeute von 86 % bezogen auf den Umsatz erzielt, der allerdings nur 50 % beträgt, denn je Mol erzeugtes Sulfenamid entsteht ein Mol Morpholinsalz des Mercaptobenzothiazols, das noch mit Natronlauge in einem separaten Arbeitsgang aufgearbeitet werden muß.

Setzt man Natronlauge zu, um das Natriumsalz des Mercaptobenzthiazols (NaMBT) als wiederverwendbares Nebenprodukt zu erzeugen und die einzusetzende Aminmenge zu reduzieren, so sinkt die Ausbeute an Sulfenamid in Abhängigkeit von der Natronlaugemenge bis auf 10 % ab.

Aufgabe der Erfindung ist es, ein ökonomisches Verfahren zur Herstellung von reineren Benzothiazolsulfenamiden in verbesserter Ausbeute und mit verbesserter Lagerstabilität bereitzustellen.

Überraschenderweise wurde nun gefunden, daß die Umsetzung von MBTS mit primären oder sekundären Aminen in Abwesenheit von Oxidationsmitteln in einem mit Wasser nicht mischbaren organischen Lösungsmittel, in dem das erzeugte Sulfenamid löslich ist, und in Gegenwart von wäßrigem Alkali in hoher Ausbeute zu reinen Benzothiazolylsulfenamiden dann gelingt, wenn bei der Reaktionsführung bestimmte Bedingungen eingehalten werden.

Gegenstand der Erfindung ist daher ein Verfahren zur Herstellung von Benzothiazolsulfenamiden aus Dibenzothiazoldisulfid und einem primären oder sekundären Amin in Abwesenheit eines Oxidationsmittels und in Anwesenheit von wäßrigem Alkali, dadurch gekennzeichnet, daß man entweder eine Lösung des Amins in einem mit Wasser nicht mischbaren organischen Lösungsmittel vorlegt und Dibenzothiazolyldisulfid und wäßriges Alkali entweder gleichzeitig oder nacheinander zugibt oder das Dibenzothiazolyldisulfid in einem mit Wasser nicht mischbaren organischen Lösungsmittel suspendiert, dann das Amin und anschließend das wäßrige Alkali zugibt, wobei jeweils das wäßrige Alkali so zugibt, daß sich ein $p_H$ von 10 bis 13, vorzugsweise von 11 bis 12, einstellt. Die Reaktion wird bei einer Temperatur von 0 bis 80 °C, vorzugsweise 20 bis 60 °C, durchgeführt.

Die beiden flüssigen Phasen werden getrennt. Durch Eindampfen der organischen Phase erhält man das Sulfenamid und gewinnt dabei Lösungsmittel und Amin zurück.

Aus der wäßrigen Phase werden überschüssiges Amin (durch Destillation) und Mercaptobenzothiazol (durch Fällung mit Säure) zurückgewonnen.

Als wäßriges Alkali kommen insbesondere Natronlauge und Kalilauge in Frage.

Geeignete Lösungsmittel sind z. B. Toluol und Xylol.

Als Amine kommen insbesondere n-Propylamin, Isopropylamin, n-Butylamin, tert.-Butylamin, Pentylamine, Cyclopentylamin, Cyclohexylamin, Dimethylamin, Diethylamin, Morpholin, Piperazin, Pyrrolidin und Piperidin in Frage.

Das erfindungsgemäße Verfahren ist insbesondere bei Verwendung von Cyclohexylamin, Isopropylamin, tert.-Butylamin und Morpholin wertvoll.

Das Verfahren kann sowohl diskontinuierlich als auch kontinuierlich durchgeführt werden.

Beispiel 1

4-(2-Benzothiazolylthio)-morpholin

In einem 2 l-Mehrhalskolben mit Rührer, Tropftrichter, Kühler, Thermometer und pH-Elektrode wurden 435 g Morpholin und 1 000 g Toluol vorgelegt. Bei 20-30 °C wurden 332 g Dibenzothiazolyldisulfid in kleinen Portionen innerhalb von 20-30 Minuten eingetragen. Parallel dazu tropfte man 200 g 20 gew.-%ige Natronlauge zu, so daß der pH zwischen 10 und 13 lag. Nach beendeter Zugabe rührte man bis zu 1 Stunde nach, trennte beide Phasen und schüttelte die wäßrige Phase mit 500 g Toluol aus. Die vereinigten organischen Phasen ergaben nach Eindampfung 4-(2-Benzothiazolylthio)-morpholin in 99,5 %iger Ausbeute. Das Produkt hatte einen Wirkstoffgehalt von 99 %, einen Gehalt an freiem Amin von 0,1 %, einen Gehalt an in Methanol unlöslichen Bestandteilen von 0 % und einen Schmelzpunkt von 85,5-87 °C bei einer Aufheizrate von 1 °C/min. Diese Werte blieben nach 24-stündiger Alterung in wasserfeuchter Atmosphäre unverändert.

Beispiel 2

60 g Cyclohexylamin wurden in 455 g Toluol gelöst. Zu der Lösung wurden nacheinander 100 g Dibenzothiazolyldisulfid und 12,1 g NaOH, gelöst in 170 g Wasser, zugegeben. Nach 10 Minuten wurde die Toluolphase von der wäßrigen Phase getrennt, mit Wasser gewaschen und im Vakuum bei 65 °C eingedampft.

Man erhielt 79,5 g N-Cyclohexylbenzothiazolsulfenamid (100 % d. Th.) mit einem Gehalt von 97 %. Der Gehalt an freiem Amin betrug 1 %.

Die wäßrige Phase wurde angesäuert, das ausgefällte MBT abfiltriert und getrocknet. Man erhielt 45,9 g (91,2 % d. Th.) mit einem Gehalt von 97,4 %.

## Patentansprüche

1. Verfahren zur Herstellung von Benzthiazolsulfenamiden aus Dibenzothiazolyldisulfid und einem primären oder sekundären Amin in Abwesenheit eines Oxidationsmittels und in Anwesenheit von wäßrigem Alkali, dadurch gekennzeichnet, daß man entweder eine Lösung des Amins in einem mit Wasser nicht mischbaren organischen Lösungsmittel vorlegt und Dibenzothiazolyldisulfid und wäßriges Alkali entweder gleichzeitig oder nacheinander zugibt oder das Dibenzothiazolyldisulfid in einem mit Wasser nicht mischbaren organischen Lösungsmittel suspendiert, dann das Amin und anschließend das wäßrige Alkali zugibt, wobei man jeweils das wäßrige Alkali so zugibt, daß sich ein $p_H$ von 10 bis 13 einstellt.

2. Verfahren nach Anspruch 1, dadurch gekennzeichnet, daß man das wäßrige Alkali so zugibt, daß sich ein $p_H$ von 11 bis 12 einstellt.

3. Verfahren nach Ansprüchen 1 und 2, dadurch gekennzeichnet, daß man die Reaktion bei 0 bis 80 °C durchführt.

4. Verfahren nach Anspruch 3, dadurch gekennzeichnet, daß man die Reaktion bei 20 bis 60 °C durchführt.

5. Verfahren nach Ansprüchen 1-4, dadurch gekennzeichnet, daß man als Amin Cyclohexylamin, Isopropylamin, tert.-Butylamin oder Morpholin einsetzt.

## Claims

1. Process for the preparation of benzothiazole sulphenamides from benzothiazolyl disulphide and a primary or secondary amine in the absence of an oxidizing agent in the presence of aqueous alkali, characterised in that either a solution of the amine in a water-immiscible organic solvent is introduced into the reaction vessel and dibenzothiazolyl disulphide and aqueous alkali are added either simultaneously or successively or the dibenzothiazolyl disulphide is suspended in a water-immiscible organic solvent and the amine is the added, followed by the aqueous alkali, the aqueous alkali being added in such a manner that a pH of from 10 to 13 established.

2. Process according to claim 1, characterised in that the aqueous alkali is added in such a manner that a pH of from 11 to 12 is established.

3. Process according to claims 1 and 2, characterised in that the reaction is carried out at 0 to 80 °C.

4. Process according to claim 3, characterised in that the reaction is carried out at 20 to 60 °C.

5. Process according to claims 1 to 4, characterised in that the amine used is cyclohexylamine, isopropylamine, tert.-butylamine or morpholine.

## Revendications

1. Procédé de préparation de benzothiazolesulfénamides à partir du disulfure de dibenzothiazolyle et d'une amine primaire ou secondaire, en l'absence d'agent oxydant et en présence d'un alcali aqueux, caractérisé en ce que l'on ajoute simultanément ou successivement du disulfure de dibenzothiazolyle et un alcali aqueux à une solution de l'amine dans un solvant organique non miscible à l'eau, ou bien on met le disulfure de dibenzothiazolyle en suspension dans un solvant organique non miscible à l'eau puis on ajoute l'amine et ensuite l'alcali aqueux, en ajoutant dans chaque cas l'alcali aqueux de manière à régler à un pH de 10 à 13.

2. Procédé selon la revendication 1, caractérisé en ce que l'on ajoute l'alcali aqueux en quantité suffisante pour régler à un pH de 11 à 12.

3. Procédé selon les revendications 1 et 2, caractérisé en ce que l'on effectue la réaction à une température de 0 à 80 °C.

4. Procédé selon la revendication 3, caractérisé en ce que l'on effectue la réaction à une température de 20 à 60 °C.

5. Procédé selon les revendications 1 à 4, caractérisé en ce que l'on utilise en tant qu'amine la cyclohexylamine, l'isopropylamine, la tert.-butylamine ou la morpholine.